(19) Europäisches Patentamt
European Patent Office
Office européen des brevets

(11) Numéro de publication: **0 176 438**
**B1**

# (12) FASCICULE DE BREVET EUROPÉEN

(45) Date de publication du fascicule du brevet: **25.11.87**

(51) Int. Cl.⁴: **C 07 C 149/18**, C 07 C 149/00

(21) Numéro de dépôt: **85401826.4**

(22) Date de dépôt: **19.09.85**

(54) **Nouveaux hydroxy-thia-alcènes, leur procédé de préparation et applications.**

(30) Priorité: **25.09.84 FR 8414665**

(43) Date de publication de la demande:
**02.04.86 Bulletin 86/14**

(45) Mention de la délivrance du brevet:
**25.11.87 Bulletin 87/48**

(84) Etats contractants désignés:
**CH DE GB LI NL SE**

(56) Documents cité:
**GB-A-441 457**
**US-A-3 591 475**

(73) Titulaire: **SOCIETE NATIONALE ELF AQUITAINE Société anonyme dite, Tour Elf 2, Place de la Coupole La Défense 6, F-92400 Courbevoie (FR)**

(72) Inventeur: **Labat, Yves, 35 bis rue Hounau, F-64000 Pau (FR)**

(74) Mandataire: **Kohn, Armand, 5 Avenue Foch, F-92380 Garches (FR)**

**EP 0 176 438 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un nouveau type d'hydroxythia alcènes, c'est-à-dire des alcools thioéthers aliphatiques, dont la chaîne comporte une ou plusieurs doubles liaisons. Un procédé de préparation de ces composés fait également partie de l'invention.

Des alcools thioéthers porteurs d'une double liaison en bout de chaîne, de forme générale $HO\text{-}CH_2\text{-}CH_2\text{-}S\text{-}P\text{-}CH=CH_2$, dans laquelle P est un alkylène comportant de 4 à 6 atomes de carbone, notamment l'hydroxy-1 thia-3 nonène-8 $HO(CH_2)_2\text{-}S\text{-}(CH_2)_4\text{-}CH=CH_2$, sont connus et utilisés. L'obtention de ce composé nécessite l'emploi de l'hexadiène-1,5 et de mercapto éthanol pour effectuer la réaction

$$HO\text{-}C_2H_4\text{-}SH + CH_2=CH\text{-}CH_2\text{-}CH_2\text{-}CH=CH_2 \longrightarrow$$

$$HO\text{-}C_2H_4\text{-}S(CH_2)_4\text{-}CH=CH_2$$

Or, l'hexadiène-1,5 est un hydrocarbure relativement coûteux et la réaction susindiquée se fait avec un rendement en produit insaturé ne dépassant pas, pratiquement, 80 %.

La présente invention apporte un progrès sensible à la technique rappelée ci-dessus: elle réalise la synthèse d'un nouveau type d'alcool-thioéthers insaturés, dont le rendement en produits insaturés est bien amélioré, pratiquement quantitatif, et qui permet d'utiliser des matières premières beaucoup plus accessibles que dans la technique antérieure. Ce procédé perfectionné permet l'obtention de composés dont la chaîne peut comporter plusieurs doubles liaisons.

L'invention est basée sur la découverte assez étonnante que les diènes conjugués réagissent avec des mercapto-alcools avec des rendements en produits insaturés plus élevés que lorsqu'on utilise les diènes non conjugués. Ainsi trouvet-on que l'hexadiène - 2,4 se combine au mercapto-éthanol avec un rendement en produits insaturés dépassant 95 %, alors que celui-ci ne dépasse guère 80 %, dans le cas de l'hexadiène-1,5.

Le nouveau procédé, suivant l'invention, consiste donc à faire réagir un mercapto-alcool sur un diène en présence d'un catalyseur radicalaire, et il est caractérisé en ce que les doubles liaisons du diène sont conjuguées.

Ainsi, les diènes, utilisés suivant l'invention, répondent- ils à la formule

$$R^1CH=\underset{\displaystyle R^3}{\underset{|}{C}}-\underset{\displaystyle R^4}{\underset{|}{C}}=CHR^2,$$

où $R^1$ à $R^4$ son des groupes aliphatiques, semblables ou différents, ou/et des atomes d'hydrogène. Le composé le plus simple, mais aussi le plus économique, est le butadiène, c'est-à-dire le diène dans lequel tou les symboles $R^1$ à $R^4$ représentent des atomes d'hydrogène. Un autre diène, fort intéressant, est l'isoprène, soit méthyl-2 butadiène-1,3,dans lequel $R^1$, $R^2$ et $R^4$ sont des H, tandis que $R^3$ est un méthyle; la ramification, constituée par celui-ci, peut apporter des modifications avantageuses aux propriétés des produits fabriqués à partir de l'alcool-thioéther correspondant.

D'autres diènes, convenant à la réalisation de l'invention, sont, à titre non limitatif, le pipérylène, c'est-à-dire le pentadiène-1,3, l'éthyl-2 butadiène-1,3, l'hexadiène-1,3 ou 2,4, l'octadiène-1,3 ou 3,5, le limonène, etc.

Différents mercapto-alcools conviennent à la réalisation de l'invention. Pour simplifier, on lés désigne ici par la formule générale $HO\text{-}(CH_2)_n\text{-}SH$, mais il est bien entendu que la chaîne ou le groupe aliphatique $(CH_2)_n$ peut comporter des ramifications ou/et divers substituants en particulier $CH_3$.

Le plus souvent, n est un nombre entier de 1 à 12, ce qui correspond à des composés allant du mercapto-méthanol à un alcool mercapto-laurique; cependant, pour la pratique courante, suffisent généralement les composés dont $n=2$ à 6, c'est-à-dire les alcools mercaptoéthylique, propylique, isopropylique, butylique, isobutylique, tert.butylique, etc.

Les catalyseurs radicalaires sont bien connus, on n'en cite donc que quelques-uns ici: azo-bis-isobutyronitrile, peroxyde de benzoyle, perbenzoate de tert.butyle, peroxyde de lauroyle, peroxyde d'isobutyryle, peroxy-acétate de tert-butyle, peroxyde de di-cumyle, hydroxy-peroxyde-p.menthane. On peut également utiliser des techniques photochimiques.

La température à laquelle le diène se combine le plus avantageusement avec le mercapto-alcool dépend de la nature de ces réactifs et de celle du catalyseur employé; elle est généralement comprise entre 0 et 100° C et, de préférence, entre 50 et 90° C; elle est liée à la température de décomposition du peroxyde.

Un facteur important du nouveau procédé est la proportion molaire du diène utilisé par rapport au mercapto-alcool. Il s'avère, en effet, que selon le rapport molaire diène/mercapto-alcool, on peut obtenir une gamme d'alcool-thioéthers non saturés, dont la chaîne comporte le reste d'une ou de plusieurs molécules de diène par molécule de mercapto-alcool. C'est là un des caractères surprenants de l'invention, qui apporte un résultat fort intéressant, en ce qu'il devient possible de préparer, à partir des matières premières, des mélanges de composés utiles, insaturés. Pour des composés difonctionnels, les compositions de différents alcool-thioéthers insaturés, pouvant ainsi être synthétisées, sont indiquées plus loin: on note ici seulement que le nouveau procédé peut être pratiqué avec des proportions de 1 à 10 molécules de diène pour 1 molécule de mercapto-

alcool, les rapports préférés étant de 1,5 à 6 molécules du premier pour 1 du second.

Les nouveaux alcool-thioéthers insaturés, suivant l'invention, peuvent être représentés schématiquement par la formule générale:

$$HO-(CH_2)_n-S\left[\underset{\underset{R}{|}}{-CH}-CH=CH-(CH_2)_m-CH_2\right]_p H \qquad (1)$$

Dans cette formule n définit la chaîne ou le groupe aliphatique du mercapto-alcool; sa valeur est de 1 à 10 et, de préférence, de 2 à 6 comme déjà indiqué plus haut; de même des ramifications ou/et substitutions peuvent exister sur $(CH_2)_n$ · R est un H ou une ramification alkylique, dont le nombre d'atomes de carbone ne dépasse généralement pas 5: il dépend de la nature du diène utilisé. m, qui dépend également de la longueur de la chaîne varie en général de 0 à 6; p donne la mesure de la polymérisation du diène sur la molécule d'alcool-thioéther. Ce coefficient dépend du rapport diène/mercapto-alcool mentionné plus haut; il est d'autant plus grand que ce rapport est élevé, notamment 1 à 10 et surtout 1 à 3.

Ce qui vient d'être exposé est illustré ci-après par le cas du butadiène réagissant, à titre d'exemple, avec du mercapto-éthanol; la réaction d'une molécule du premier avec une du second donne:

$HSCH_2CH_2OH + CH_2=CH-CH=CH_2 \rightarrow HOCH_2CH_2-S-CH_2-CH=CH-CH_3$ (2)
hydroxy-1 thia-3 heptène-5(HTH)

Ce composé est désigné par la suite par l'abréviation "HTH"; il correspond à la formule (1) dans laquelle n=2, R=H, m=O, p=I.

Le second composé, qui se forme à côté du précédent, surtout lorsque le nombre de moles de butadiène dépasse 2 pour 1 de mercapto-éthanol, est

$HOCH_2CH_2S-CH_2-CH=CH-CH_2-CH_2-CH=CH-CH_3$ (3)
hydroxy-1 thia-3 undécadiène (HTU)

Il résulte de l'addition d'une mole de butadiène au composé précédent HTH; n, R et m sont les mêmes que plus haut, mais p=2.

Une troisième série d'alcools-thioéthers insaturés se forme dans la réaction suivant l'invention, surtout lorsqu'il y a plus de 3 moles de butadiène par mole de mercapto-éthanol; ce sont des produits renfermant plus de 2 doubles liaisons; il s'agit particulièrement de

$HOCH_2CH_2S-CH_2-CH=CH-CH_2-CH_2-CH=CH-CH_2-CH_2-CH=CH-CH_3$(HTP) (4)
hydroxy-1 thia-3 pentadécatriène-5,9,13

Ce triène ou ses isomères se forment en proportions croissantes lorsque le rapport butadiène/mercapto-éthanol augmente, surtout au-delà de 3. Ici les coefficients, n, m et R sont les mêmes que plus haut, mais p=3.

Des isomères de ces trois composés existent, mais on peut affirmer que la structure des produits ci-dessus est largement prédominante. Ainsi y-a-t-il, en particulier, des isomères à terminaison vinylique, identifiés par analyse RMN, tels que:

$HO-(CH_2)_2-S-CH_2-CH_2-CH=CH_2$ environ 5% par rapport à HTH

$HO-(CH_2)_2-S-(CH_2)_2-\underset{\underset{CH_3}{|}}{C}=CH-CH_2-CH=CH_2$ " 20% " " " HTU

$HO-(CH_2)_2-S-CH_2-CH=CH-CH_2-CH_2-CH_2-\underset{\underset{CH_3}{|}}{C}=CH-\underset{\underset{\underset{\underset{CH_2}{\|}}{CH}}{|}}{CH_2}$ en mélange avec HTP.

HTH, HTU et HTP peuvent être séparés après distillation des mélanges avec leurs isomères. Cela peut être réalisé notamment dans les conditions suivantes:

| | Température °C | Vide |
|---|---|---|
| HTH | 70 | 1 mm Hg |
| HTU | 105 | 1 mm Hg |
| HTP | 175 | 0,7 mm Hg |

Cette variété de structure des alcools-thioéthers, qu'il est possible d'obtenir suivant l'invention, permet de faire varier les propriétés des produits d'applications de ces composés. Pour cela, l'invention prévoit la modification du rapport molaire diène/mercapto-alcool et, éventuellement, la séparation des produits obtenus, comme exposé plus haut, cependant qu'une utilisation de ces produits bruts constitue une forme de réalisation intéressante.

Avec des diènes autres que le butadiène, les choses se passent de la même façon, mais naturellement, le nombre d'isomères possibles est plus grand. A titre d'exemple on mentionne, ci-après, le premier composé, hydroxy-1 thia-3 éthyl-4 nonène-5.

$$HO-CH_2CH_2-S-\underset{\displaystyle C_2H_5}{\underset{|}{CH}}-CH=CH-CH_2CH_2CH_3$$

qui s'obtient à partir de l'octadiène-3,5 et du mercapto-éthanol; par rapport à la formule (1) on a ici $n=2$, $R=C_2H_5$, $m=2$, $p=1$.

Les exemples non limitatifs qui suivent illustrent l'invention.

**Exemple 1**

Dans un autoclave de 20 l, muni d'un agitateur et d'un thermostat, on introduit 6458 g de mercaptoéthanol $HO\text{-}CH_2\text{-}CH_2\text{-}SH$, soit 82,8 moles, 6707 g de butadiène $CH_2=CH\text{-}CH=CH_2$, c'est-àdire 124,2 moles, 150 g d'azo-bis-isobutyronitrile, comme catalyseur.

Ainsi, le rapport molaire butadiène/mercaptoéthanol est égal à 1,5. Le mélange est chauffé à 80°C pendant 10 h, après quoi on élimine le butadiène restant.

Les 12744 g de mélange réactionnel se composent de:

70 %, soit 67 moles de HTH (abréviation adoptée aux pages précédentes),
20,5 %, soit 13,8 moles de HTU,
4 %, soit 2 moles de HTP,
5,5 % de divers.

Le rendement par rapport au mercaptoéthanol est pratiquement quantitatif. Le mélange réactionnel obtenu contient environ 9 fonctions insaturées et 6,6 fonctions alcool par kg de produit, confirmé par des analyses RMN et l'analyse chimique. Ces fonctions insaturées sont totalement réactives visà-vis des composés à fonction mercaptan.

**Exemple 2**

On opère comme dans l'exemple 1 mais aves les quantités suivantes de réactifs: 3300 g de mercapto propanol, soit 35,8 moles, 5730 g de butadiène, soit 106 moles, 150 g d'azo-bis-isobutyronitrile.

Le rapport molaire butadiène/mercapto-propanol est égal à 2,95.

Le mélange réactionnel obtenu contient environ 10,2 fonctions insaturées et 6,8 fonctions alcool par kg de produit.

**Exemple 3**

Essais comparatifs avec un diène non conjugué

Les opérations de l'exemple 1 sont répétées, mais le butadiène est remplacé par le même nombre de moles d'hexadiène-1,5. On obtient alors principalement de l'hydroxy-1 thia-3 nonène-8 (désigné par les initiales HTN dans la suite de la description). Mais le rendement par rapport au mercaptoéthanol n'est que 80 %.

**Exemples 4 à 8**

Suivant la technique de l'exemple 1, on effectue une série de préparations avec des rapports molaires butadiène/mercaptoéthanol croissants. Les conversion et rendement sont calculés par rapport au mercaptoéthanol.

On peut voir que les conversions vont de 93 à 98,5 %. L'idée inventive est confirmée par le fait que la teneur en produits plus lourds augmente avec l'excès de butadiène utilisé. Les 3 dernières lignes horizontales du tableau ci-après montre bien qu'en faisant varier l'excès de butadiène, on peut modifier la composition des alcools thioéthers obtenus assez profondément.

**Tableau**

| Exemples | 4 | 5 | 6 | 7 | 8 |
|---|---|---|---|---|---|
| Rapport molaire Butadiène/mercaptoéthanol | 1,5 | 3 | 3 | 6 | 6 |
| Procédé (*) | D | D | C | D | C |
| Conversion % | 98,5 | 98 | 95 | 98,4 | 93 |
| Rendement % HTH | 81,7 | 56 | 51 | 46 | 32 |
| Rendement % HTU | 12 | 25 | 26,7 | 25,6 | 25,7 |
| Composition % HTH | 75,3 | 40,7 | 36 | 27,5 | 19,7 |
| Composition % HTU | 15,8 | 25 | 26,7 | 21,5 | 22,1 |
| Produits plus lourds | 9 | 34,3 | 37 | 51 | 58,2 |

*) D = discontinu; C = continu.

**Revendications**

1. Procédé de préparation d'un alcool thioéther insaturé par l'action d'un mercaptoalcool en $C_1$ à $C_{12}$ sur un diène, caractérisé en ce que le diène est conjugué, répondant à la formule

$$R^1CH{=}\underset{\displaystyle R^3}{\underset{|}{C}}-\underset{\displaystyle R^4}{\underset{|}{C}}{=}CHR^2$$

où $R^1$ à $R^4$ sont des groupes aliphatiques, semblables ou différents, ou/et des atomes d'hydrogène.

2. Procédé suivant la revendication 1, caractérisé en ce que le diéne est le butadiène.

3. Procédé suivant la revendication 1, caractérisé en ce que le diène est l'isoprène, le pentadiène-1,3, l'éthyl-2 butadiène-1,3, l'hexadiène-1,3 ou 2,4, l'octadiène-1,3 ou 3,5 ou le limonène.

4. Procédé suivant une des revendications 1 à 3, caractérisé en ce que le mercaptoalcool est du type $HO(CH_2)_n$ SH, la chaîne ou le groupe aliphatique $-(CH_2)_n$ - pouvant porter des substitutions ou/et des ramifications.

5. Procédé suivant la revendication 4, caractérisé en ce que n est 2 à 6.

6. Procédé suivant une des revendications 1 à 5, caractérisé en ce que l'on fait varier le rapport molaire diène/mercaptoalcool entre 1 et 10, et de préférence entre 1,5 et 6 selon la longueur de chaîne et le nombre de doubles liaisons voulues pour l'alcool thioéther à préparer.

7. Nouveau produit chimique constitué par un alcool thioéther insaturé, caractérisé par la structure

$$HO-(CH_2)_nS\left[-\underset{\displaystyle R}{\underset{|}{C}H}-CH{=}CH-(CH_2)_m-CH_2\right]_p H$$

où n est un entier de 1 à 10, R est H ou un alkyle de préférence ne dépassant pas $C_5$, m a une valeur de 0 à 6, tandis que p peut varier entre 1 et 10 et surtout de 1 à 3, étant entendu que, si m = O, R n'est pas H ou/et p est supérieur à 1.

8. Nouveau produit suivant la revendication 7, caractérisé en ce qu'il est constitué par de l'hydroxy-1 thia-3 undecadiène-5,9 ou/et de l'hydroxy-1 thia-3 pentadécatriène-5,9, 13.

9. Nouveau produit suivant la revendication 7 ou 8, caractérisé en ce qu'il contient des alcools thioéthers terminés par un groupe vinylique.

**Patentansprüche**

1. Verfahren zur Herstellung eines ungesättigten Thioetheralkohols durch Einwirkung eines Mercaptoalkohols mit 1 bis 12 C-Atomen auf ein Dien, dadurch gekennzeichnet, dass das Dien konjugiert ist und der Formel

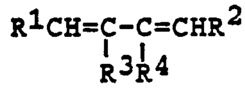

entspricht, worin $R^1$ bis $R^4$ gleiche oder verschiedene aliphatische Gruppen oder/und Wasserstoffatome bedeuten.

2. Verfahren nach Anspruch 1, dadurch gekennzeichnet, dass es sich beim Dien um Butadien handelt.

3. Verfahren nach Anspruch 1, dadurch gekennzeichnet, dass es sich beim Dien um Isopren, 1,3-Pentadien, 2-Ethyl-1,3-butadien, 1,3- oder 2,4-Hexadien, 1,3- oder 3,5-Octadien oder Limonen handelt.

4. Verfahren nach einem der Ansprüche 1 bis 3, dadurch gekennzeichnet, dass der Mercaptoalkohol dem Typ $HO(CH_2)_nSH$ angehört, wobei die aliphatische Kette oder Gruppe $-(CH_2)_n-$Substitutionen oder/und Verzweigungen aufweisen kann.

5.Verfahren nach Anspruch 4, dadurch gekennzeichnet, dass n 2 - 6 bedeutet.

8. Verfahren nach einem der Ansprüche 1 bis 5, dadurch gekennzeichnet, dass man das Molverhältnis Dien/Mercaptoalkohol von 1 bis 10 und vorzugsweise von 1,5 bis 6 je nach der Länge der Kette und der Anzahl der Doppelbindungen, die im herzustellenden Thioetheralkohol beabsichtigt sind, variiert.

7. Neues chemisches Produkt, bestehend aus einem ungesättigten Thioetheralkohol, gekennzeichnet durch die Struktur

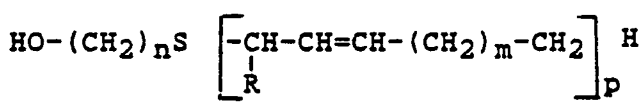

wobei n eine ganze Zahl von 1 bis 10 ist, R H oder ein Alkyl mit vorzugsweise nicht mehr als 5 C-Atomen ist, m einen Wert von 0 bis 6 hat, während p von 1 bis 10 und insbesondere von 1 bis 3 variieren kann, mit der Massgabe, dass wenn m = O, R nicht H ist oder/und p grösser als 1 ist.

8. Neues Produkt nach Anspruch 7, dadurch gekennzeichnet, dass es aus 1-Hydroxy-3-thia-5,9-undecadien oder/und 1-Hydroxy-3-thia-5,9,13-pentadecatrien besteht.

9. Neues chemisches Produkt nach Anspruch 7 oder 8, dadurch gekennzeichnet, dass es Thioetheralkohole mit einer endständigen Vinylgruppe enthält.

**Claims**

1. Process of preparation of an unsaturated thioether-alcohol by the action of a $C_1$ to $C_{12}$ mercapto-alcohol on a diene, characterised in that the diene is conjugated and has the formula

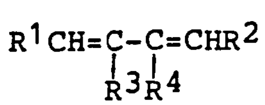

where $R^1$ to $R^4$ are like or different aliphatic groups and/or hydrogen atoms.

2. Process according to claim 1, characterised in that the diene is butadiene.

3. Process according to claim 1, characterised in that the diene is isoprene, 1,3-pentadiene, 2-ethyl-1,3-butadiene, 1,3 or 2,4-hexadiene, 1,3 or 3,5-octadiene or limonene.

4. Process according to any of claims 1 to 3, characterised in that the mercapto-alcohol is of the type $HO(CH_2)_nSH$, the aliphatic chain or group $-(CH_2)_n-$ optionally carrying substituents and/or sidechains.

5. Process according to claim 4, characterised in that n is 2 to 6.

8. Process according to any of claims 1 to 5, characterised in that the diene/mercapto-alcohol molar ratio is varied between 1 and 10, and preferably between 1.5 and 6, depending upon the length of chain and the number of double bonds desired in the thioether-alcohol being prepared.

7. New chemical product constituted by an unsaturated thioether-alcohol, characterised by the structure

$$HO-(CH_2)_nS\ [-\underset{R}{CH}-CH=CH-(CH_2)_m-CH_2]_p\ H$$

where n is an integer from 1 to 10, R is H or an alkyl group, preferably not exceeding $C_5$, m has a value of 0 to 6, while p can vary between 1 and 10 and particularly from 1 to 3, provided always that, if m = O, R is not H and/or p is greater than 1.

8. New product according to claim 7, characterised in that it is constituted by 1-hydroxy-3-thia 5,9-undecadiene and/or by 1-hydroxy-3-thia-5,9,13-pentadecatriene.

9. New product according to claim 7 or 8, characterised in that it contains thioether-alcohols terminated by a vinyl group.